# EUROPEAN PATENT APPLICATION

(11) **EP 4 617 382 A1**
(43) Date of publication of application: **17.09.2025**
(21) Application number: 22967656.4
(22) Date of filing: 09.12.2022
(51) Int. Cl.: C12Q 1/6869, C12Q 1/6806, C12Q 1/6844

(54) **SYNTHESIS METHOD FOR SECOND STRAND IN DNB PAIRED-END SEQUENCING, SEQUENCING METHOD, AND RELATED PRODUCTS**

(71) Applicant: MGI Tech Co., Ltd., Shenzhen, Guangdong 518083 (CN)
(72) Inventor: XU, Chongjun, Shenzhen, Guangdong 518083 (CN); JIANG, Lan, Shenzhen, Guangdong 518083 (CN); YANG, Jin, Shenzhen, Guangdong 518083 (CN); GONG, Meihua, Shenzhen, Guangdong 518083 (CN)
(74) Representative: Zacco GmbH
(86) International application number: PCT/CN2022/138100
(87) International publication number: WO 2024/119509

(57) **Abstract**

The present invention provides a synthesis method for a second strand in DNB paired-end sequencing, a sequencing method and a related product. The synthesis method for the second strand comprises: after a first-strand sequencing on the DNB is completed, performing a second-strand synthesis using a second-strand synthesis primer, wherein the second-strand synthesis primer is a primer capable of being anchored on the surface of a sequencing chip. Second-strand synthesis is performed by the second-strand synthesis primer anchored to a surface of a sequencing carrier, so that the process of the second-strand synthesis is relatively more stable, and the synthesized second-strand template strand is anchored to the sequencing carrier and subsequently the second-strand template strand anchored to the sequencing carrier is sequenced, so that a relatively higher quality of the second-strand sequencing can be obtained.

## Description

### Technical Field

The present invention relates to the field of gene sequencing technologies, and in particular, to a synthesis method for a second strand in DNB(DNA nanoball) paired-end sequencing, a sequencing method and a related product.

### Background

Paired-end sequencing based on DNBSEQ sequencing platform refers to, when a DNA library to be tested is constructed, adding sequencing primer binding sites to linkers at both ends, performing the first round of sequencing, then removing the template strand of the first round of sequencing by means of a strand replacement reaction, forming a template used in the second round of sequencing, and then performing a second round of synthesis sequencing of a complementary strand. When analyzing the sequencing quality of the second-strand sequencing, it was found that the second-strand sequencing quality was poor, mainly manifested as: high Lag%, low Q30%, fast signal decrease, etc. How to obtain better quality of second-strand sequencing is of great significance to the improvement of the overall sequencing quality.

The reaction of generating a second strand by means of the strand displacement activity of Phi29 polymerase is a process that requires very fine control, for example, there is need to control the stability of Phi29 DNA polymerase. The Phi29 polymerase has a poor stability, and any decrease in enzyme activity may affect the synthesis of the second strand, resulting in high second-strand Lag%, and then a low Q value and fast signal decrease. In addition, in order to effectively remove the Phi29 DNA polymerase, a novel elution reagent needs to be screened so as to increase the elution time. However, the described two aspects are not beneficial for high-efficiency sequencing.

After the reaction of generating the second strand, the residual Phi29 DNA polymerase, the blocking of ddNTPs, and DNA products after excessive strand displacement will all cause the quality of the second strand sequencing to decrease. How to make the second-strand sequencing like the first-strand sequencing and avoid using the strand displacement reaction to obtain excess second-strand templates is a key step to improve the quality of second-strand sequencing.

### Summary

The main object of the present invention is to provide a synthesis method for a second-strand in DNB paired-end sequencing, a sequencing method and a related product, so as to solve the problem of poor quality of second-strand sequencing in the prior art.

In order to achieve the described object, according to one aspect of the present invention, provided is a synthesis method for a second-strand in a DNB paired-end sequencing. The synthesis method comprises: performing first-strand sequencing on a DNB, and then synthesizing a second-strand using a second-strand synthesis primer, wherein the second-strand synthesis primer is a primer capable of being anchored on a surface of a sequencing chip.

Further, an end of the second-strand synthesis primer has a modification, and is conjugated with a bridge molecule on the surface of the sequencing chip by means of the modification, and preferably, the end of the second-strand synthesis primer has a DBCO modification, an SH modification, an azido modification or an alkyne modification; and preferably, the bridge molecule on the surface of the sequencing chip is NHS-PEG8-azido, SMCC, NHS-DBCO, or NHS-azido.

Further, the described synthesis method comprises: after the first-strand sequencing is completed, eluting and removing a first-strand sequencing strand bound to the DNB to release the DNB; using the released DNB as a template, and using the second-strand synthesis primer to perform the second-strand synthesis; preferably, at least one of the following elution reagents is used to remove the first-strand sequencing strand: an organic denaturant, a 3'-5' exonuclease and NaOH; preferably, the organic denaturant is formamide; preferably, a working concentration of formamide is of molecular biology grade, and more preferably, a mass concentration is 100-99.5%; and preferably, a mass concentration of NaOH is 0.01-0.1 M.

Further, the described synthesis method uses a Bst3.0 polymerase for the second-strand synthesis; and preferably, after the second-strand synthesis, the synthesis method further comprises: digesting and removing DNB.

Further, paired-end sequencing is performed by preparing the DNB containing a digestion marker, and after the second-strand synthesis, the DNB is digested and removed using the digestion marker, wherein the digestion marker is any one selected from the following: an enzyme cutting site, a modified monodeoxyribonucleotide, an unnatural nucleotide or a ribonucleotide; preferably, the enzyme cutting site is an Nb.BbvCl enzyme cutting site: 5'-CCTCAGC-3'; preferably, the modified monodeoxyribonucleotide is 8-OXO-dGTP; preferably, the unnatural nucleotide is dITP; and preferably, the ribonucleotide is dUTP.

Further, using the digestion marker to remove the DNB comprises any one of the following methods: (1) the digestion marker is an enzyme cutting site, the DNB is subjected to enzyme cutting, and then the enzyme cutting product is eluted with an organic denaturant, preferably, the DNB is subjected to enzyme cutting with Nb.BbvCl; and preferably, the organic denaturant is formamide; (2) the digestion marker is a modified monodeoxyribonucleotide, and the modified monodeoxyribonucleotide is 8-OXO-dGTP, and the DNB is digested by human alkyladenine DNA glycosylase and endonuclease IV, and then the digestion product is eluted with an organic denaturant; preferably, the digestion temperature is 37 °C; and preferably, the organic denaturant is one or more of sodium citrate, Tris-HCl and EDTA; (3) the digestion marker is an unnatural nucleotide, the unnatural nucleotide is dITP, the DNB is digested by endonuclease V, and then the digestion product is eluted with an organic denaturant; and preferably, the organic denaturant is one or more of sodium citrate, Tris-HCl and EDTA; and (4) the digestion marker is a ribonucleotide, the ribonucleotide is dUTP, the DNB is digested by USER enzyme, and then the digestion product is eluted with an organic denaturant; and preferably, the organic denaturant is one or more of sodium citrate, Tris-HCl and EDTA.

In order to achieve the described object, according to one aspect of the present invention, provided is a second-strand sequencing method in a DNB paired-end sequencing. The sequencing method comprises: using the described second-strand synthesis method to synthesize a second-strand sequencing template; and sequencing the second-strand sequencing template using a second-strand sequencing primer.

In order to achieve the described object, according to one aspect of the present invention, provided is a sequencing chip, the surface of the sequencing chip is modified with a bridge molecule capable of being conjugated with a primer having a terminal modification, wherein the bridge molecule is any one selected from the following: NHS-PEG8-azido, SMCC, NHS-DBCO, and NHS-azido.

In order to achieve the described object, according to one aspect of the present invention, provided is a kit comprising any more of the following: Nb.BbvCl enzyme, human alkyladenine DNA glycosylase, endonuclease IV, endonuclease V, USER enzyme, formamide, a DBCO-bearing primer, an SH-bearing primer, an azido-bearing primer, an alkynyl-bearing primer and Bst3.0 polymerase.

In order to achieve the described object, according to one aspect of the present invention, provided is a sequencing complex. The sequencing complex comprises the sequencing chip, wherein the sequencing chip carries DNB, an optional first-strand sequencing strand, a second-strand synthesis primer or a second-strand sequencing template strand, and the second-strand synthesis primer or the second-strand sequencing template strand is fixed on the sequencing chip.

Further, a bridge molecule is modified on the surface of the sequencing chip in the described sequencing complex, a coupling group is modified on the second-strand synthesis primer, and the second-strand synthesis primer is fixed on the sequencing chip by means of coupling between the coupling group and the bridge molecule; preferably, an end of the second-strand synthesis primer has a DBCO modification, an SH modification, an azido modification, or an alkynyl modification; and preferably, the bridge molecule on the surface of the sequencing chip is NHS-PEG8-azido, SMCC, NHS-DBCO, or NHS-azido.

In order to achieve the described object, according to one aspect of the present invention, provided is a DNB paired-end sequencing method. The sequencing method comprises: loading the DNB on the described sequencing chip; performing the first-strand sequencing on the DNB, and then performing the described second-strand sequencing method; or performing the first-strand sequencing on the DNB; after the first-strand sequencing is completed, using the described method to perform the second-strand synthesis to obtain a second-strand sequencing template; and performing the second-strand sequencing on the second-strand sequencing template.

By applying the technical solution of the present invention, when second-strand synthesis is performed, a second-strand synthesis primer anchored to the surface of a sequencing carrier is used to perform the second-strand synthesis, so that the process of the second-strand synthesis is relatively more stable, and the synthesized second-strand template strand is anchored to the sequencing carrier and subsequently the second-strand template strand anchored to the sequencing carrier is sequenced, so that a relatively higher quality of the second-strand sequencing can be obtained.

### Brief Description of the Drawings

The accompanying drawings, which form a part of the present application, are used to provide a further understanding of the present invention. The schematic examples of the present invention and the description thereof are used to explain the present invention, and do not form improper limits to the present invention. In the drawings:
Fig. 1 shows a technical solution route according to the invention.
Fig. 2 shows a schematic of conventional DNB sequencing.

### Detailed Description of the Embodiments

It is important to note that the examples of the present disclosure and the characteristics in the examples can be combined under the condition of no conflicts. Hereinafter, the present invention will be described in detail with reference to examples.

As mentioned in the background art, second-strand sequencing results of the existing DNB paired-end sequencing method show high Lag%, low Q30%, fast signal decrease, etc., so that the quality of the second-strand sequencing is poor. Moreover, polymerase Phi29 used in the second-strand synthesis has a problem of insufficient stability, and is also one of factors affecting the quality of the second-strand sequencing. Therefore, in the present application, the inventor adopts a novel second-strand synthesis method. When performing second-strand synthesis, the second-strand synthesis is performed by a second-strand synthesis primer anchored on the surface of a sequencing carrier, so as to ensure that the second-strand synthesis process is carried out in an orderly manner by the primer anchored on the sequencing carrier, and high-quality second-strand sequencing is performed under the condition of stable second-strand synthesis. In addition, the quality assurance of second-strand sequencing can also be achieved by using a Bst3.0 polymerase having higher stability to synthesize the second-strand.

In a first typical embodiment of the present invention, provided is a second-strand synthesis method in a DNB paired-end sequencing. The synthesis method comprises: after first-strand sequencing on a DNB is completed, performing second-strand synthesis using a second-strand synthesis primer; wherein the second-strand synthesis primer is a primer capable of being anchored to a surface of a sequencing carrier.

The described synthesis method of the present invention differs from the conventional second-strand synthesis method in that, in the present invention, the second-strand synthesis primer is anchored to the surface of the sequencing carrier. Conventional second-strand synthesis uses a free primer to perform second-strand synthesis. However, in the present application, a second-strand synthesis primer is fixed, so that a synthesized second-strand template strand is fixed on the surface of a sequencing carrier to form a stable second-strand template strand, so as to improve the quality of subsequent sequencing using the second-strand template strand.

In the present application, in order to further improve the stability of the second-strand sequencing template on the carrier, the idea of anchoring the second-strand to the sequencing carrier is used. By anchoring the second-strand synthesis primer to a sequencing chip, the second-strand synthesis process is more stable to produce a high quality second-strand template strand. Specifically, the method of anchoring the second-strand sequencing template onto the sequencing carrier through a synthesis step may be implemented by a known chemical coupling method. An end of the described second-strand synthesis primer has a modification, and is conjugated with a bridge molecule on the surface of the sequencing chip by means of the modification, and any chemical substance that can achieve coupling can be used as a terminal modification and a bridge substance. In a preferred example, the end of the second-strand synthesis primer has a DBCO modification, an SH modification, an azido modification, or an alkynyl modification; and in a preferred example, the bridge molecule on the surface of the sequencing chip is NHS-PEG8-azido, SMCC (full name: 4-(N-maleimidomethyl)cyclohexane-1-carboxylic acid succinimide ester), NHS-DBCO (full name of DBCO is dibenzocyclooctyne), or NHS-azido.

In the described second-strand synthesis method, performing second-strand synthesis by taking DNB as a template further comprises: after the first-strand sequencing is completed, eluting and removing a first-strand sequencing strands bound to the DNB to release the DNB; and using the released DNB as a template, and using the second-strand synthesis primer to perform second-strand synthesis. There are various methods for eluting and removing the first-strand sequencing strand. The present application includes, but is not limited to, removing the first-strand sequencing strand by using at least one of the following elution reagents: an organic denaturant, a 3'-5' exonuclease, and NaOH. In a preferred example, the organic denaturant is formamide. In a preferred example, a working concentration of formamide is 100%-99.5%, molecular biology grade. In a preferred example, a mass concentration of NaOH is from 0.01 to 0.1 M. In order to release the DNB, it is necessary to destroy the double helix structure formed by linking between the first-strand sequencing strand and the DNB. Both a strong base and an organic denaturant can destroy hydrogen bonds between the double helices of the DNA, the 3'-5' exonuclease degrades the DNA by destroying phosphodiester bonds, and the first-strand sequencing strand is further removed by elution; when it is eluted using formamide, the conditions are as follows: the temperature being 40°C for 10 minutes, under which the DNB will not be eluted. In addition, as DNB is single-stranded DNA, is in the shape of a wool ball, and has no 3'-5' free end, it will not be degraded.

In order to further improve the purity of the synthesized second-strand template so as to further reduce or avoid the subsequent interference of DNB in the application of the second-strand template, in a preferred example of the present application, after the described second-strand synthesis, the synthesis method further comprises: digesting and removing the DNB. After the second-strand synthesis is completed, the DNB template is digested and removed, so that only the synthesized second-strand exists when the second-strand template strand is applied subsequently (for example, in a system of second-strand sequencing), thereby further avoiding the problem in the prior art that sequencing quality deteriorates because too many second-strand templates exist in the system during second-strand sequencing. In a preferred example, the enzyme used in the second-strand synthesis is a Bst3.0 polymerase. The second-strand is generated by using a stable DNA polymerase with high heat resistance, so as to improve the stability of the second-strand synthesis strand, thereby further ensuring the quality of the second-strand sequencing.

The described method for digesting and removing DNB is not limited, and any method capable of achieving this purpose is suitable for the present application. In some preferred examples of the application, the DNB containing a digestion marker is prepared for paired-end sequencing, and the DNB is digested and removed by the digestion marker after the second-strand synthesizing. The digestion marker is any one selected from an enzyme cutting site, a modified monodeoxyribonucleotide, an unnatural nucleotide, or a ribonucleotide. By destroying the DNA double helix structure of the DNB, the DNB is fragmented and the digestion of the DNB is completed.

The digestion marker of the enzyme cutting site can be subjected to an enzyme cutting treatment by a related enzyme. In a preferred example, the enzyme cutting site is an Nb.BbvCl enzyme cutting site: 5'-CCTCAGC-3'; the modified monodeoxyribonucleotide can also be identified and cut by a specific repair enzyme, and in a preferred example, the modified monodeoxyribonucleotide is 8-OXO-dGTP; in a preferred example, the unnatural nucleotide is dITP; and in a preferred example, the ribonucleotide is dUTP.

Digesting and removing the DNB by the described digestion marker includes any one of the following methods: (1) the DNB is subjected to enzyme cutting, and then an enzyme cutting product is eluted with an organic denaturant, and in a preferred example, enzyme cutting is performed with Nb. BbvCl enzyme, and the organic denaturant is formamide; (2) the DNB is digested by human alkyladenine DNA glycosylase and endonuclease IV followed by elution of a digestion product with an organic denaturant; in a preferred example, the DNB comprises 8-OXO-dGTP site; the digestion temperature is 37 °C; and the organic denaturant is one or more of sodium citrate, Tris-HCl, and EDTA; (3) endonuclease V is used to digest the DNB, and then an organic denaturant is used to elute a digestion product; in a preferred example, the DNB comprises dITP; the organic denaturant is one or more of sodium citrate, Tris-HCl, and EDTA; (4) the DNB is digested using USER Enzyme followed by elution of a digestion product with an organic denaturant; in a preferred example, the DNB comprises dUTP; and the organic denaturant is one or more of sodium citrate, Tris-HCl, and EDTA.

When the DNB contains a modified monodeoxyribonucleotide, i.e. 8-OXO-dGTP, the DNB is digested with hAAG (human alkyladenine DNA glycosylase) and endonuclease IV. After the two enzymes recognize the 8-OXO-dGTP site, hAAG cleaves the phosphodiester bond at the 5' end of the 8-OXO-dGTP site to produce a 3' hydroxyl and a 5' deoxyribose phosphate end; moreover, the endonuclease IV also has 3' diesterase activity and 3'-5' exonuclease activity, and the removal of the DNB (first-strand sequencing strand) is completed subsequently by elution.

When the DNB contains unnatural nucleotide dITP, the DNB is digested by the endonuclease V. After identifying the dITP site, the enzyme cleaves the second phosphodiester bond at the 3' end of the site to form 3' hydroxyl and 5' phosphate, and then the DNB is removed by elution.

When the DNB contains unnatural nucleotide dUTP, the DNB is digested by USER enzyme. The USER is a mixture of uracil DNA glycosylase (UDG) and DNA glycosylase-lyase Endo VIII. UDG catalyzes the cleavage of uracil bases, forming an abasic (apyrimidinic) site but leaving the phosphodiester backbone intact. The cleavage activity of Endo VIII enables phosphodiester bonds at 3' and 5' ends of abasic sites to be broken, releasing abasic deoxyribose; and subsequent removal of DNB is completed by elution.

In a second typical embodiment of the present invention, provided is a sequencing method for second-strand sequencing in a DNB paired-end sequencing. The sequencing method comprises: using the described second-strand synthesis method to synthesize a second-strand sequencing template; and sequencing the second-strand sequencing template by a second-strand sequencing primer. The second-strand synthesis strand obtained by the described second-strand synthesis method has a better quality under the condition of a more stable DNA polymerase, and after the DNB elution in the system, the interference of the DNB is eliminated, which can provide a powerful guarantee for improving the quality of the second-strand sequencing to the maximum extent.

In a third typical embodiment of the present invention, provided is a sequencing chip, and the surface of the sequencing chip is modified with a bridge molecule capable of being conjugated with a primer having a terminal modification, wherein the bridge molecule is any one selected from: NHS-PEG8-azido, SMCC, NHS-DBCO, and NHS-azido. By modifying the bridge molecule on the surface of the sequencing chip, the sequencing chip ensures that a primer for synthesis of a second-strand can be anchored to the chip, so that the process of the synthesis of the second-strand is more stable, providing quality assurance support for subsequent second-strand synthesis strand sequencing.

In a fourth exemplary embodiment of the present invention, provided is kit. The kit comprises any one or more of: Nb.BbvCl enzyme, human alkyladenine DNA glycosylase, endonuclease IV, endonuclease V, USER enzyme, formamide, a DBCO-bearing primer, an SH-bearing primer, an azido-bearing primer, an alkynyl-bearing primer, and Bst3.0 polymerase. The kit comprises relevant substances in the second-strand synthesis process of the present invention to ensure the digestion and elution in the second-strand synthesis process and maximize the quality improvement of the second-strand synthesis process.

In a fifth typical embodiment of the present invention, provided is a sequencing complex. The sequencing complex comprises a sequencing chip. The sequencing chip carries DNB, an optional first-strand sequencing strand, and a second-strand synthesis primer or a second-strand sequencing template strand, wherein the second-strand synthesis primer or the second-strand sequencing template strand is fixed on the sequencing chip. In a preferred example, a bridge molecule is modified on the surface of the sequencing chip, a coupling group is modified on the second-strand synthesis primer, and the second-strand synthesis primer is fixed on the sequencing chip through coupling between the coupling group and the bridge molecule. In a preferred example, an end of the second-strand synthesis primer has a DBCO modification, an SH modification, an azido modification, or an alkynyl modification; and the bridge molecule on the surface of the sequencing chip is NHS-PEG8-azido, SMCC, NHS-DBCO, or NHS-azido. This sequencing support can be used for second-strand synthesis, and by fixing the second-strand template strand on the solid support, a stable environment is provided for the second-strand synthesis process, which is beneficial to improve the quality of its synthesis and sequencing.

In a sixth exemplary embodiment of the present invention, provided is a DNB paired-end sequencing method. The sequencing method comprises: loading the DNB onto the described sequencing chip; performing the first-strand sequencing on the DNB, and then performing the described second-strand sequencing method; or performing the first-strand sequencing on the DNB; after the first-strand sequencing is completed, using the described method to perform the second-strand synthesis to obtain a second-strand sequencing template; and performing the second-strand sequencing on the second-strand sequencing template. By means of the described DNB paired-end sequencing method, a second-strand synthesis strand with better quality can be obtained, and after the DNB is eluted in the system, the interference of DNB is eliminated, which can provide a strong guarantee for the quality of second-strand sequencing to the greatest extent.

The present disclosure will be further described in detail with reference to the following examples, which should not be construed as limiting the scope of protection of the present disclosure.

### Example 1

Experimental devices used in this example were a BGISEQ-500 sequencer, a MGIDL-200H loader, and a BGISEQ-500 sequencing slide.

The experimental reagents and raw materials used in this example were: Formamide (analytically pure), NHS-PEG8-azido, ultrapure water, E. coli single-stranded circular DNA as a template, i.e. standard library reagent V3.0, primer sequence: CAACTCCTTGGCTCACAGAACATGGCTACGATCCGACTT (SEQ ID NO: 1). DNA polymerase was from BGI, and DNA nanospheres were derived from BGI. The following experiments all used E. coli single-stranded circular DNA as a template, and used a BGISEQ-500 high throughput sequencing kit to complete preparation of the DNA nanospheres and load same onto a chip for subsequent sequencing.

The digestion marker sequence mentioned in this example was an Nb. BbvCl enzyme cutting site, i.e. 5'-CCTCAGC-3'; after the first-stand sequencing was completed, a new synthetic strand was eluted with a formamide reagent; DBCO-MP1 primer was conjugated to the surface of the sequencing slide under certain conditions with NHS-PEG8-azido as a bridge.

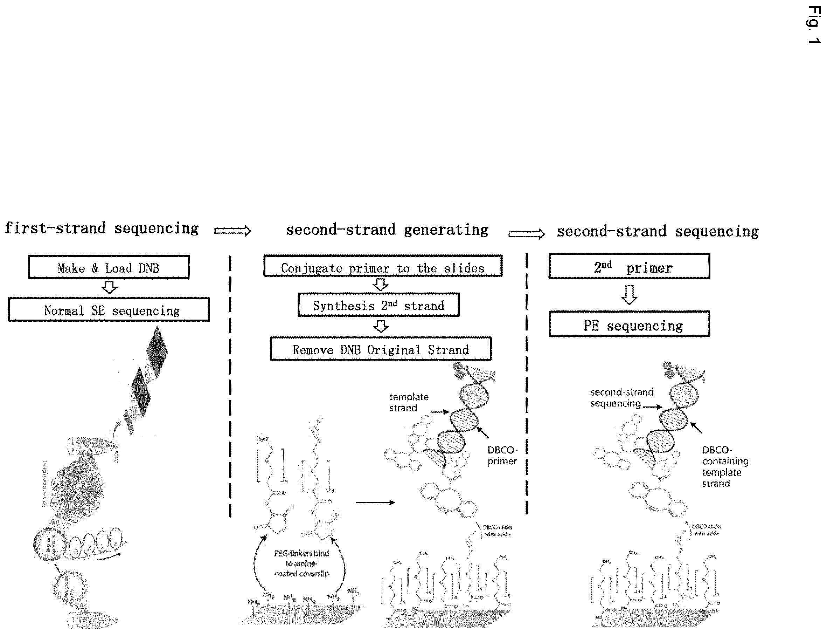

The specific method comprised: dissolving the NHS-PEG8-azido bridge in PBS at a certain concentration (0.001-1 mg/mL), processing same in the sequencing slide at room temperature for 30 min, and eluting the unreacted NHS-PEG8-azido reagent by PBS; dissolving the DBCO-MP1 primer in a primer hybridization buffer solution (with a composition of 5 x SSC) to a certain concentration (0.1-2 µM), processing same in a sequencing slide at 40 °C for 3 h, processing the sequencing slide in formamide at 37 °C for 10 min, and washing the unreacted DBCO-MP1 primer; and then pumping Bst3.0 polymerase for second-strand synthesis (as shown in figure 1).

### Comparative Example 1

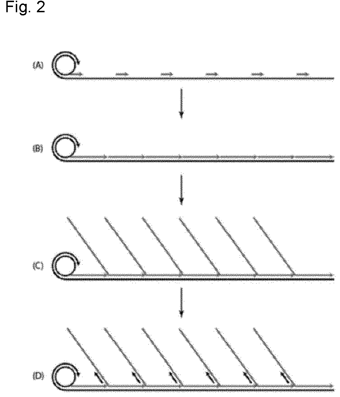

Comparative Example 1 differs from Example 1 in that the synthesis was performed by conventional second-strand synthesis (as shown in figure 2). That is, NHS-PEG8-azido was not used as a bridge to conjugate the DBCO-MP1 primer to the surface of the sequencing slide.

The sequencing quality results of Example 1 and Comparative Example 1 were compared:
The test conditions of Example 1: Primer: Read 1: the primer was normally inserted; Read 2: bridge molecule NHS-PEG8-azido conjugated the DBCO-primer to the surface of the sequencing chip to initiate second-strand synthesis: L02: Bst3.0 @ 57 °C, 30 min.

The test conditions of comparative example 1: Read 1: the primer was normally inserted; L02: normal paired-end sequencing method, i.e. performing second-strand sequencing by using the product of read 1 as a second-strand sequencing template strand.

**Table 1:**

| | Example 1 | Comparative Example 1 |
|---|---|---|
| Total reads | 179.02 | 151.06 |
| Q30% | 91.77 | 88.3 |
| ESR% | 82.09 | 69.68 |
| Lag% | 0.09 | 0.12 |

From the description above, it can be determined that the described examples of the present invention achieve the following technical effects: the second-strand sequencing results of the method of the present invention can obtain higher Total Reactions, Q30% and ESR% and lower second-strand Lag%. These indexes are of great significance for NGS sequencing: Total Reads represents the total number of reads contained in the fastq file generated by basecall without splitting, and the higher, the better; Q30% represents the proportion of bases with an estimated error rate lower than 0.001 (accuracy higher than 99.9%) in the basecal results, and the higher, the better; ESR% represents the proportion of valid reads in the original but unfiltered fastq data generated by the basal treatment, and the higher, the better; and the Lag% index can be used to evaluate the degree of completeness of a biochemical reaction, and the lower, the better. It can be determined therefrom that the use of such solution and reagents in the sequencing method and the sequencing kit will improve the sequencing quality of a product, thereby contributing to the improvement of the value and market share of the product.

The description above is only the preferred examples of the present invention, and is not intended to limit the present invention. For a person skilled in the art, the present invention may have various modifications and variations. Any modifications, equivalent replacements, improvements and the like made within the spirit and principle of the present invention shall fall within the protection scope of the present invention.

## Claims

1. A method for synthesizing a second-strand in a DNB paired-end sequencing, wherein the method comprises:
upon completion of a first-strand sequencing on a DNB, synthesizing the second-strand using a second-strand synthesis primer, wherein the second-strand synthesis primer is an anchoring primer on a surface of a sequencing chip.

2. The method according to claim 1, wherein an end of the second-strand synthesis primer has a modification, and is conjugated with a bridge molecule on the surface of the sequencing chip through the modification,
preferably, the end of the second-strand synthesis primer has a DBCO modification, an SH modification, an azido modification, or an alkyne modification; and
preferably, the bridge molecule on the surface of the sequencing chip is NHS-PEG8-azido, SMCC, NHS-DBCO, or NHS-azido.

3. The method according to claim 1, wherein the method comprises: upon completion of the first-strand sequencing on the DNB, eluting and removing a first-strand sequencing strand bound to the DNB to release the DNB; and using the released DNB as a template, and synthesizing the second-strand using the second-strand synthesis primer;
preferably, eluting and removing the first-strand sequencing strand with at least one of the following elution reagents: an organic denaturant, a 3'-5' exonuclease, and NaOH;
preferably, the organic denaturant is formamide;
preferably, a mass concentration of the formamide is 100-99.5%; and
preferably, a mass concentration of the NaOH is 0.01-0.1 M.

4. The method according to claim 1, wherein synthesizing the second-strand using a Bst3.0 polymerase; and
preferably, upon synthesis of the second-strand, the method further comprises digesting and removing the DNB.

5. The method according to claim 4, wherein, performing the paired-end sequencing by preparing the DNB with a digestion marker, and after the second-strand synthesis, using the digestion marker to digest and remove the DNB,
wherein the digestion marker is any one selected from: an enzyme cutting site, a modified monodeoxyribonucleotide, an unnatural nucleotide, or a ribonucleotide;
preferably, the enzyme cutting site is Nb. BbvCl enzyme cutting site: 5'-CCTCAGC-3'; preferably, the modified monodeoxyribonucleotide is 8-OXO-dGTP;
preferably, the unnatural nucleotide is dITP; and
preferably, the ribonucleotide is dUTP.

6. The method according to claim 5, wherein using the digestion marker to digest and remove the DNB comprises any one of the following methods:
(1) the digestion marker is the enzyme cutting site, cleaving the DNB with an enzyme, and then eluting an enzyme cutting product with an organic denaturant,
preferably, performing the enzyme cutting with Nb. BbvCl enzyme; and
preferably, the organic denaturant is formamide;
(2) the digestion marker is the modified monodeoxyribonucleotide, and the modified monodeoxyribonucleotide is 8-OXO-dGTP, digesting the DNB with human alkyladenine DNA glycosylase and endonuclease IV, and then eluting a digestion product with an organic denaturant;
preferably, the digestion temperature is 37 °C; and
preferably, the organic denaturant is one or more of sodium citrate, Tris-HCl, and EDTA;
(3) the digestion marker is the unnatural nucleotide, and the unnatural nucleotide is dITP, digesting the DNB with endonuclease V, and then eluting a digestion product with an organic denaturant; and
preferably, the organic denaturant is one or more of sodium citrate, Tris-HCl, and EDTA; and
(4) the digestion marker is the ribonucleotide, and the ribonucleotide is dUTP, digesting the DNB with USER enzyme, and then eluting a digestion product with an organic denaturant; and preferably, the organic denaturant is one or more of sodium citrate, Tris-HCl, and EDTA.

7. A method for sequencing a second-strand in a DNB paired-end sequencing, wherein the method comprises:
synthesizing a second-strand sequencing template by the method for synthesising a second-strand according to any one of claims 1 to 6; and
sequencing the second-strand sequencing template by a second-strand sequencing primer.

8. A sequencing chip, wherein a bridge molecule capable of being conjugated with a primer having a terminal modification is modified on the surface of the sequencing chip, wherein the bridge molecule is any one selected from: NHS-PEG8-azido, SMCC, NHS-DBCO, and NHS-azido.

9. A kit, wherein the kit comprises any more of: Nb.BbvCl enzyme, human alkyladenine DNA glycosylase, endonuclease IV, endonuclease V, USER enzyme, formamide, a DBCO-bearing primer, an SH-bearing primer, an azido-bearing primer, an alkynyl-bearing primer, and Bst3.0 polymerase.

10. A sequencing complex, wherein the sequencing complex comprises a sequencing chip, the sequencing chip carries DNB, an optional first-strand sequencing strand, and a second-strand synthesis primer or a second-strand sequencing template strand, wherein the second-strand synthesis primer or the second-strand sequencing template strand is fixed on the sequencing chip.

11. The sequencing complex according to claim 10, wherein a bridge molecule is modified on the surface of the sequencing chip, a coupling group is modified on the second-strand synthesis primer, and the second-strand synthesis primer is fixed on the sequencing chip by a coupling between the coupling group and the bridge molecule;
preferably, an end of the second-strand synthesis primer has a DBCO modification, an SH modification, an azido modification, or an alkyne modification; and
preferably, the bridge molecule on the surface of the sequencing chip is NHS-PEG8-azido, SMCC, NHS-DBCO, or NHS-azido.

12. A method for a DNB paired-end sequencing, wherein the method comprises:
loading the DNB on the sequencing chip of claim 8;
performing the first-strand sequencing on the DNB, and then performing the second-strand sequencing by the method of claim 7;
or
performing the first-strand sequencing on the DNB;
upon completion of the first-strand sequencing, performing the second-strand synthesis by the method of any one of claims 1 to 6 to obtain a second-strand sequencing template; and performing the second-strand sequencing on the second-strand sequencing template.
